# EUROPÄISCHE PATENTANMELDUNG

(11) **EP 1 674 055 A1**
(43) Veröffentlichungstag der Anmeldung: **28.06.2006**
(21) Anmeldenummer: 05020648.1
(22) Anmeldetag: 22.09.2005
(51) Int. Cl.: A61F 5/11

(54) **Vorrichtung zur Vornahme von Nagelkorrekturen**

(30) Priorität: 23.11.2004 DE 102004056382
(71) Anmelder: Bernd Stolz GmbH, 92224 Amberg (DE)
(72) Erfinder: Stolz, Bernd, 92224 Amberg (DE)
(74) Vertreter: Schneck, Herbert

(57) **Zusammenfassung**

Bei einer Vorrichtung zur Vornahme von Nagelkorrekturen, insbesondere bei eingewachsenen, zu stark gekrümmten Nägeln, umfassend einen Streifen aus eigenelastischem Kunststoff, der mittels eines Schnellklebers längs seiner gesamten Länge mit dem zu korrigierenden Nagel verklebt werden kann, ist vorgesehen, dass die Außenränder (5) des Streifens (4) sich keilförmig nach außen verjüngend ausgebildet sind.

## Beschreibung

Die Erfindung betrifft eine Vorrichtung zur Vornahme von Nagelkorrekturen, insbesondere bei eingewachsenen, zu stark gekrümmten Nägeln, umfassend einen Streifen aus eigenelastischem Kunststoff, der mittels eines Schnellklebers längs seiner gesamten Länge mit dem zu korrigierenden Nagel verklebt werden kann.

Eine derartige Vorrichtung ist beispielsweise aus DE 37 08 811 A1 bekannt und hat sich in der Praxis außerordentlich bewährt.

Der Erfindung liegt die Aufgabe zu Grunde, eine Vorrichtung der gattungsgemäßen Art so weiterzubilden, dass sowohl die Handhabung noch weiter verbessert wird als auch noch günstigere Eigenschaften im aufgeklebten Zustand erreicht werden.

Diese Aufgabe wird erfindungsgemäß dadurch gelöst, dass die Außenränder des Streifens sich keilförmig nach außen verjüngend ausgebildet sind.

Durch diese keilförmige Verjüngung wird im Randbereich die Rückstellkraft etwas reduziert, was häufig wünschenswert ist bzw. als angenehm empfunden wird, und gleichzeitig werden Kanten vermieden und ein weicher Übergang erreicht, so dass beim Anziehen von Strümpfen oder dergleichen keine Probleme auftreten.

In weiterer Ausgestaltung der Erfindung ist vorgesehen, dass wenigstens ein Streifen vor der Applikation auf einen Träger angeordnet ist, insbesondere auf einem Silikon-Träger, der elastisch verformbar ist.

Hierdurch ist es beim Applizieren des Streifens möglich, den elastischen Träger so zu verformen, dass der darauf befindliche Streifen etwas angehoben wird und dementsprechend leicht zu erfassen.

Dies wird auch dadurch unterstützt, dass der Träger aus einer Grundplatte und einer Mehrzahl von Erhebungen besteht, wobei auf der Oberseite jeder Erhebung ein Streifen angeordnet ist, der dementsprechend leichter von benachbarten Streifen bei der Verarbeitung separiert werden kann.

Weiterhin kann mit Vorteil vorgesehen sein, dass die Länge der Erhebungen wenigstens auf einer Seite größer ist als die Länge des Streifens, so dass im überstehenden Bereich der Erhebung diese beim Abheben des Streifens vom Streifen weggebogen werden kann.

Nachfolgend wird die Erfindung an Hand eines bevorzugten Ausführungsbeispiels in Verbindung mit der Zeichnung näher beschrieben. Dabei zeigen:
- Fig. 1: einen Längsschnitt durch eine erfindungsgemäße Vorrichtung zur Vornahme von Nagelkorrekturen,
- Fig. 2: eine vergrößerte Darstellung des Bereichs II in Fig. 1, und
- Fig. 3: einen Schnitt durch einen applizierten Streifen.

Eine in der Zeichnung dargestellte Vorrichtung zur Vornahme von Nagelkorrekturen umfasst einen plattenartigen Silikonträger 2, welcher eine Mehrzahl voneinander beabstandeter, parallel zueinander verlaufender Erhebungen 3 aufweist. Auf der Oberseite jeder Erhebung 3 ist ein Kunststoff-Streifen 4 angeordnet, der sich nicht über die gesamte Länge der Erhebung 3 erstreckt, d. h. ein Teil der Erhebung 3 (in der Zeichnung rechts außen) ist nicht von dem Streifen bedeckt. Zur Applikation eines Kunststoff-Streifens 4 auf einem Zehennagel 6 wird die Oberseite des auf dem Silikonträger 2 befindlichen, zu applizierenden Kunststoff-Streifens 4 mit Schnellkleber bestrichen, der Kunststoff-Streifen 4 wird dann zusammen mit dem Träger 2 auf die Oberseite des Zehennagels 6 aufgedrückt, und nach dem Aushärten des Klebers wird dann der Träger 2 vom aufgeklebten Kunststoff-Streifen 4 abgezogen, wobei dieses Abziehen dadurch erleichtert wird, dass ein Teil der Erhebung 3 nicht von dem Kunststoff-Streifen 4 bedeckt ist.

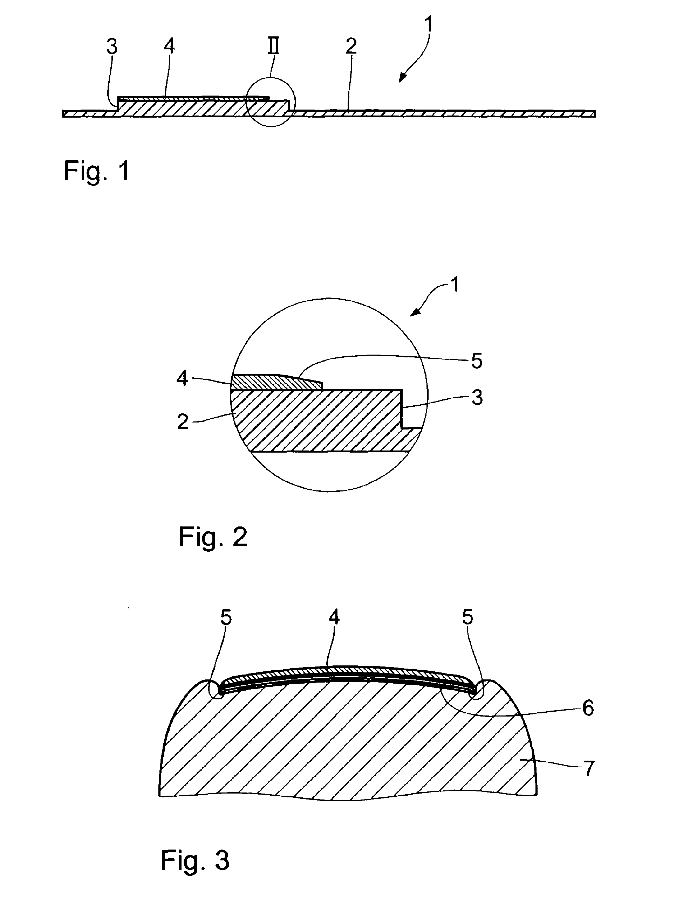

Die äußeren Ränder 5 bzw. die Stirnseiten des Streifens 4 sind sich keilförmig nach außen verjüngend ausgebildet, wie es insbesondere in Fig. 2 erkennbar ist.

In Fig. 3 ist ein Streifen 4 im applizierten Zustand dargestellt, d. h. der Streifen ist quer über einen Zehennagel 6 einer Zehe 7 aufgeklebt, wobei die Ränder 5 auf Grund ihrer keilförmigen Ausgestaltung zum Nagel 6 hin verlaufend ausgebildet sind.

## Patentansprüche

1. Vorrichtung zur Vornahme von Nagelkorrekturen, insbesondere bei eingewachsenen, zu stark gekrümmten Nägeln, umfassend einen Streifen aus eigenelastischem Kunststoff, der mittels eines Schnellklebers längs seiner gesamten Länge mit dem zu korrigierenden Nagel verklebt werden kann, **dadurch gekennzeichnet, dass** die Außenränder (5) des Streifens (4) sich keilförmig nach außen verjüngend ausgebildet sind.

2. Vorrichtung nach Anspruch 1, **dadurch gekennzeichnet, dass** wenigstens ein Streifen (4) vor der Applikation auf einem Träger (1) angeordnet ist, insbesondere auf einem Silikon-Träger (1), der elastisch verformbar ist.

3. Vorrichtung nach Anspruch 2, **dadurch gekennzeichnet, dass** der Träger (1) aus einer Grundplatte (2) und einer Mehrzahl von Erhebungen (3) besteht, wobei auf der Oberseite jeder Erhebung ein Streifen (4) angeordnet ist.

4. Vorrichtung nach Anspruch 3, **dadurch gekennzeichnet, dass** die Länge der Erhebungen (3) wenigstens auf einer Seite größer ist als die Länge des Streifens (4).
